# EUROPEAN PATENT APPLICATION

(11) **EP 2 241 543 A1**
(43) Date of publication of application: **20.10.2010**
(21) Application number: 09425143.6
(22) Date of filing: 17.04.2009
(51) Int. Cl.: C07C 29/147, C07C 33/02, C07C 51/02, C07C 51/16, C07C 51/41, C07C 57/03

(54) **Process for the preparation of 2,4,6-octatriene-1-oic acid and 2,4,6-octatriene-1-ol**

(71) Applicant: Giuliani S.p.A., 20129 Milano (IT)
(72) Inventor: Giuliani, Giammaria, 20123 Milano (IT); Benedusi, Anna, 20124 Milano (IT); Milanese, Alberto, 20052 Monza (IT)
(74) Representative: Appoloni, Romano

(57) **Abstract**

The present invention concerns a process for synthesis of 2,4,6-octatriene-1-oic acid and 2,4,6-octatriene-1-ol, which comprises the following stages:
a) reaction between 2,4-trans-hexadienal and triethyl phosphonoacetate to give ethyl 2,4,6-trans-octatrienoate;
b) alkaline hydrolysis of ethyl 2,4,6-trans-octatrienoate to give the corresponding alkaline salt;
c) acidification of said salt to give 2,4,6-trans-octatrienoic acid, which can be separated or can undergo the following further stages:
d) reaction of the 2,4,6-trans-octatrienoic acid with ethyl chloroformiate to give the mixed anhydride formed by 2,4,6-trans-octatrienoic acid and ethyl carbonic acid;
e) reduction of said mixed anhydride with sodium borohydride to give 2,4,6-transoctatrienol;

and optionally a purification stage of the end product.

## Description

The present invention concerns a new process for the synthesis of 2,4,6-octatriene-1-oic acid and 2,4,6-octatriene-1-ol.

2,4,6-octatriene-1-oic acid and 2,4,6-octatriene-1-ol are compounds of pharmaceutical interest with antioxidant properties towards free radicals, for example as described in EP1501774 held by the same Applicant, anti-inflammatory activity and inhibition of the enzyme 5-alpha-reductase involved in the activation of testosterone in numerous functional regions.

A known synthesis for obtaining said compounds is carried out by means of the corresponding aldehyde 2,4,6-octatriene-1-al. This is obtained (Kuhn, R., and Grundmann, Chem.Ber., 70, 1318 (1937)) by auto-condensation of the crotonaldehyde in the presence of pyridinium acetate as a catalyst. Said synthesis, however, does not result prevalently in one single compound but in a mixture corresponding to the various adducts deriving from the condensation of different units of crotonaldehyde. Thus, for example, in order to obtain 2,4,6-octatrienal, a mixture of aldehydes is produced with 8 atoms of carbon (2,4,6-octatriene-1-al), 12 atoms of carbon (2,4,6,8,10-dodecapentaen-1-al) and 16 atoms of carbon (2,4,6,8,10,12,14-hexadecaheptaen-1-al) deriving respectively from the condensation of 2, 3 or 4 units of crotonaldehyde, in addition to numerous other reaction by-products. These compounds are difficult to separate, hence the final yields, after isolation and complete purification of the compound required, are extremely low, in the order of 2-3%.

The present invention proposes a new synthesis method for preparing the compounds described, substantially facilitating production on an industrial scale accompanied by a high yield and purity of the end product.

For said purpose the invention proposes a process for the preparation of at least one compound chosen from 2,4,6-octatriene-1-oic acid and 2,4,6-octatriene-1-ol comprising the following stages:
a) reaction between 2,4-trans-hexadienal and triethyl phosphonoacetate to give ethyl 2,4,6-trans-octatrienoate;
b) alkaline hydrolysis of ethyl 2,4,6-trans-octatrienoate to give the corresponding salt;
c) acidification of said salt to give 2,4,6-trans-octatriene-1-oic acid, which can be isolated or can undergo the following further stages:
d) reaction of the 2,4,6-trans-octatrienoic acid with ethylchloroformiate to give the mixed anhydride formed by 2,4,6-trans-octatrienoic acid and ethylcarbonic acid;
e) reduction of said mixed anhydride with sodium borohydride to give 2,4,6-trans-octatrienol,
optionally followed by a final purification stage.

The process according to the invention is summarised by the following synthesis scheme, in which each compound is identified by formulas and molecular weight:

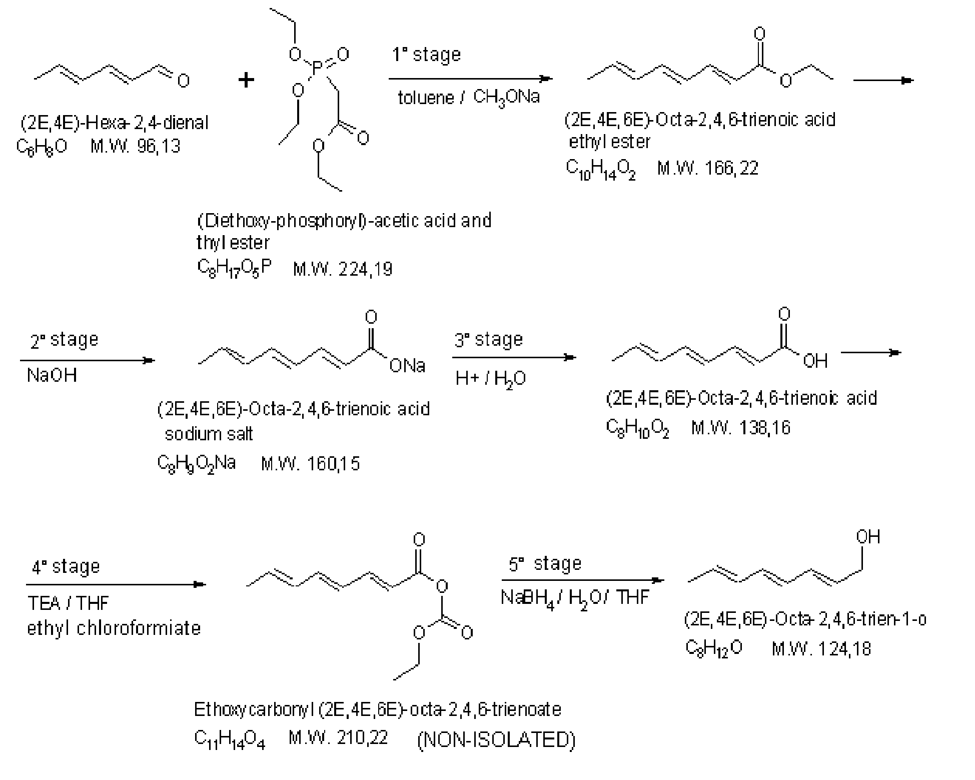

In a preferred embodiment of the invention, in the first stage a) the starting compound 2,4-trans-hexadienal, known also as sorbic aldehyde or sorbaldehyde, is condensed with triethyl phosphonoacetate, according to the Wittig-Horner scheme, in toluene and in the presence of sodium methoxide. After the normal procedures of quenching, extraction, washing and concentration, the ethyl 2,4,6-trans-octatrienoate is obtained, which can be used as is or purified by crystallisation or distillation in a vacuum. Usually the ethyl 2,4,6-trans-octatrienoate thus obtained is pure enough to be used directly.

In the second stage b), the ethyl 2,4,6-trans-octatrienoate is hydrolysed in an alkaline environment by means of sodium or potassium hydroxide in an alcoholic or hydroalcoholic solution. Normally the alkaline hydroxide is used in measured excess, at ambient temperature.

The 2,4,6-trans-octatrienoate alkaline thus obtained can be isolated by filtration or brought directly to an aqueous solution for subsequent transformation into acid.

In the third stage c), the 2,4,6-trans-octatrienoate in aqueous solution is treated with diluted mineral acids, for example hydrochloric acid, until acid pH is obtained, preferably approximately 2. The 2,4,6-trans-octatrienoic acid precipitates and can be separated by filtration from the mother liquor or by extraction with solvents immiscible with water, such as ethyl acetate or dichloromethane. This is followed by thorough drying, under a vacuum at ambient temperature if solid or on drying agents if in solution.

The 2,4,6-trans-octatriene-1-oic acid thus obtained can be isolated and used as it is as an active ingredient in the pharmaceutical field or it can undergo the following further stages to obtain 2,4,6-trans-octatriene-1-ol.

In the latter case, in the fourth stage d), the 2,4,6-trans-octatrienoic acid is placed in a solution of tetrahydrofuran (THF) and treated with triethylamine and ethyl chloroformiate, at low temperature, preferably approximately 0°C. Once the reaction has been completed, the triethylammonium hydrochloride is removed by filtration and a solution is obtained, in THF, of the mixed anhydride formed by 2,4,6-trans-octatrienoic acid and ethylcarbonic acid, which is kept at a temperature below 0°C and used within one hour.

In the fifth stage e), said solution of mixed anhydride is treated with an aqueous solution of sodium borohydride, concentrated and stabilised with soda, maintaining a temperature below +5°C. Once the reaction has been completed, following concentration and dilution with water, the raw 2,4,6-trans-octatrienol precipitates and can be separated by filtration or by extraction with solvents.

The optional conclusive purification of the raw 2,4,6-trans-octatrienol occurs by crystallisation, with saturated hydrocarbons or hydroalcoholic mixtures, preferably at temperatures below 40°C. Pure 2,4,6-trans-octatrienol is obtained, with purity higher than 95%.

In order to better understand the characteristics and advantages of the invention, non-limiting examples of practical embodiment are described below.

### Example 1

- First stage a): preparation of ethyl 2,4,6-*trans*-octatrienoate.

The following are loaded in a 2000 ml flask provided with stirrer, thermometer, reflux, dropper, cooling bath and under a strictly controlled nitrogen atmosphere:
toluene (d 0.865) 250 ml = 216.5 g and sodium methoxide solution 25% in methanol
(M.W. 54.02 d 0.945) 108.04 g = 114.33 ml = 0.5 moles.

Maintaining the temperature between 20° and 25°C (slight exotherm), the following is added dropwise:
triethyl phosphonoacetate (M.W. 224.19 d 1.13) 112.09 g = 99.2 ml = 0.5 moles.
Stirring is then performed, between 20° and 25°C, for one hour.

Maintaining the temperature between 20° and 30°C, the following is added dropwise:
sorbaldehyde > 95% (M.W. 96.13 d 0.87) 48.06 g = 55.24 ml = 0.5 moles.

The exotherm is considerable and the temperature is controlled between 20° and 25 °C.

Stirring is carried out at ambient temperature for one night (12 hours).

A qualitative TLC analytical control is performed. The qualitative TLC controls use hexane/acetone in a ratio of 7/3 as eluant.

Cooling and without exceeding 20°C, it is diluted with 1000 ml of water and with toluene 200 ml = 173 g.

It is stirred well and the phases are separated, eliminating the aqueous phase.

It is brought to a residue under a vacuum at max temperature = 40°C.

Ethyl 2,4,6-*trans*- octatrienoate is obtained (M.W. 166.22) raw (still containing solvent) 104.3 g (theoretical amount 83.11 g); quantitative yield = 0.5 moles = 100% of the theoretical amount.
- Second and third stage b) and c): preparation of 2,4,6-*trans*-octatrienoic acid.

In the same flask, the residue of ethyl 2,4,6-*trans*-octatrienoate is recovered (M.W. 166.22) wet with toluene, corresponding to 83.11 g = 0.5 moles with ethanol 95°(d 0.79) 300 ml = 237 g.

The mixture is dissolved at approximately 30°C and then, cooling and maintaining the temperature between 20 and 25°C, sodium hydroxide aqueous solution 30% is poured in (M.W. 40.0 d 1.31) 166.7 g = 127 ml = 1.25 moles = 2.5 moles/mole. It is stirred at ambient temperature for one night (12 hours).

An end-of-hydrolysis qualitative TLC analytical control is performed.

Concentration is carried out under a vacuum at 40°C. 1000 ml of deionised water are added and the mixture is dissolved at 40°C, then 10.0 g of decolouring carbon are added.

It is stirred at 40°C for one hour and filtered, washing with a little water.

It is reloaded in the same clean flask and cooled to between 0 ° and 10°C.

Without exceeding 20°C, hydrochloric acid 37% is poured in (M.W. 36.46 d 1.185) 123.18 g = 103.9 ml = 1.25 moles.

The pH is adjusted to between 0 and 2.0 and stirring is performed for 10 minutes at approximately 20°C.

It is collected in a Buchner filter, washing thoroughly with approximately 1000 ml of deionised water.

It is dried under a vacuum at max temperature = 40°C, on CaCl₂, to a residual humidity (determined by the Karl-Fischer method) of below 0.5% (approximately one night).

2,4,6-*trans*-octatrienoic acid (M.W. 138.16) 53.5 g = 0.387 moles = 77.4% of the theoretical amount is obtained.

A TLC control is performed.

### Example 2

### Fourth and fifth stage d) and e): preparation of 2,4,6-trans-octatrienol

A 3000 ml flask, equipped with stirrer, thermometer, reflux, dropper, cooling bath and with strictly controlled nitrogen atmosphere, is loaded in an anhydrous environment with 2,4,6-*trans*-octatrienoic acid (M.W. 138.16) 53.5 g = 0.387 moles, in anhydrous tetrahydrofuran (d 0.89) 800 ml = 712 g.

It is stirred at ambient temperature until complete dissolution and triethylamine is added dropwise (M.W. 101.19 d 0.73) 39.15 g = 53.6 ml = 0.387 moles.

It is cooled to 0°C and then ethyl chloroformiate is poured in (M.W. 108.52 d 1.139) 41.99 g = 36.86 ml = 0.387 moles without exceeding 15°C (there is a considerable exotherm).

It is stirred for one hour at 0°C obtaining the formation of an abundant precipitate of triethylamine hydrochloride (TEA-HCl).

Maintaining a well-controlled anhydrous environment, the TEA-HCl precipitate is filtered, pressing it well and washing it thoroughly with anhydrous tetrahydrofuran 200 ml = 178 g.

The filtrate and washing substances are re-loaded in the same flask, clean and dry, and everything is cooled to between -15° and -10°C. Slowly, controlling the violent exotherm in order not to exceed the max temperature of 0°C, paying attention to build-up and foaming, a solution of sodium borohydride (M.W. 37.8) 21.9 g = 0.58 moles = 1.5 moles/mole, cooled to approximately 0°C, is added dropwise in one/two hours to 50 ml of deionised water containing 0.2 g of sodium hydroxide.

Once pouring is complete, it is stirred for one night, leaving the temperature to rise spontaneously to 20°- 25 °C.

An end-of-reaction TLC control is performed. It is concentrated in a vacuum at max temperature 30°C, until the THF is eliminated.

The residue is recovered with 1000 ml of deionised water, stirring well.

It is cooled to 0°C and stirred again for approximately one hour.

It is collected in a Buchner filter, washing thoroughly with deionised water and pressing well.

45.5 g of wet 2,4,6-*trans*-octatrienol is obtained of adequate quality.

### Example 3

2,4,6-*trans*-octatrienol wet with water, obtained as described in example 1, is reduced to a pulp at ambient temperature, in petroleum ether, bp 60°/80°, (d 0.68) 200 ml = 136 g, for one hour.

It is collected in a Buchner filter, washing with petroleum ether 50 ml = 34 g.

It is dried in a vacuum at ambient temperature (25°C), obtaining 2,4,6-*trans-*octatrienol (M.W. 124.18) 32.0 g = 0.258 moles = 66.8% of the theoretical amount on the octatrienoic acid and 51.6% of the theoretical amount on the sorbaldehyde.

The end product purified as above undergoes NMR and HPLC analysis, which determine a purity > 95%.

As can be seen from the description as a whole and the examples given above, the process of the invention permits preparation of both the 2,4,6-trans-octatrienoic acid and the 2,4,6-octatrienol according to a synthesis method which substantially facilitates production and can be applied on an industrial scale, accompanied by a high yield and purity of the end product.

Unlike the prior art, the process of the invention avoids the use of large quantities of crotonaldehyde, an aggressive toxic raw material, with respect to the end product.

## Claims

1. Process for the preparation of at least one compound chosen from 2,4,6-octatriene-1-oic acid and 2,4,6-octatriene-1-ol, comprising the following stages:
a) reaction between 2,4-trans-hexadienal and triethyl phosphonoacetate to give ethyl 2,4,6-trans-octatrienoate;
b) alkaline hydrolysis of ethyl 2,4,6-trans-octatrienoate to give the corresponding alkaline salt;
c) acidification of said salt to give 2,4,6-trans-octatriene-1-oic acid, which can be isolated or can undergo the following further stages:
d) reaction of the 2,4,6-trans-octatrienoic acid with ethyl chloroformiate to give the mixed anhydride formed by 2,4,6-trans-octatrienoic acid and ethyl carbonic acid;
e) reduction of said mixed anhydride with sodium borohydride to give 2,4,6-trans-octatrienol,
optionally followed by a purification stage of the end product.

2. Process for the preparation of 2,4,6-trans-octatrienoic acid as claimed in claim 1 comprising the following stages:
a) reaction between 2,4-trans-hexadienal and triethyl phosphonoacetate to give ethyl 2,4,6-trans-octatrienoate;
b) alkaline hydrolysis of ethyl 2,4,6-trans-octatrienoate to give the corresponding alkaline salt;
c) acidification of said salt to give 2,4,6-trans-octatrienoic acid.

3. Process for preparation of the 2,4,6-trans-octatrienol starting from the 2,4,6-trans-octatrienoic acid obtained as claimed in claim 2, comprising the following stages:
d) reaction of the 2,4,6-trans-octatrienoic acid with ethyl chloroformiate to give the mixed anhydride formed by 2,4,6-trans-octatrienoic acid and ethyl carbonic acid;
e) reduction of said mixed anhydride with sodium borohydride to give 2,4,6-trans-octatrienol;
and optionally a final purification stage of 2,4,6-trans-octatrienol.

4. Process as claimed in claim 1 **characterised in that** in said stage a) 2,4-trans-hexadienal is condensed with triethyl phosphonoacetate in toluene and in the presence of sodium methoxide.

5. Process as claimed in claim 1 **characterised in that** in said stage b) ethyl 2,4,6-trans-octatrienoate is hydrolysed in an alkaline environment by means of sodium hydroxide in alcoholic or hydroalcoholic solution, to give sodium 2,4,6-trans-octatrienoate.

6. Process as claimed in claim 5, **characterised in that** in said stage c), the sodium 2,4,6-trans-octatrienoate is treated with mineral acids diluted in aqueous solution until obtaining acid pH and precipitation of the 2,4,6-trans-octatrienoic acid.

7. Process as claimed in claim 1 **characterised in that** in said stage d) the 2,4,6-trans-octatrienoic acid is placed in a solution of tetrahydrofuran and treated with triethylamine and ethyl chloroformiate at low temperature; once the reaction is complete, the triethylammonium hydrochloride is removed by filtration and a solution of the anhydride formed by 2,4,6-trans-octatrienoic acid and ethyl carbonic acid is obtained, kept at a maximum temperature of approximately 0°C.

8. Process as claimed in claim 1 **characterised in that** in said stage e) said mixed anhydride is treated with an aqueous solution of concentrated sodium borohydride and stabilised with soda, maintaining the temperature below 5°C until, following concentration and dilution with water, raw 2,4,6-trans-octatrienol precipitates and is separated by filtration or by extraction with solvents.

9. Process as claimed in claim 1 **characterised in that** said purification of raw 2,4,6-trans-octatrienol is performed by crystallisation with saturated hydrocarbons or hydroalcoholic mixtures.

10. Process as claimed in claim 1 **characterised in that** in said stage a) ethyl 2,4,6-trans-octatrienoate is sent as is to the subsequent stages or purified by crystallisation or distillation under a vacuum.

11. Process as claimed in claim 1 **characterised in that** in said stage b) the sodium 2,4,6-trans-octatrienoate thus obtained is isolated by filtration or brought directly to an aqueous solution for subsequent conversion into free acid.

12. 2,4,6-*trans*-octatrienol with purity higher than 95% obtained via the process of one of the preceding claims.

13. Intermediates ethyl 2,4,6-trans-octatrienoate, corresponding sodium salt, and mixed anhydride formed by 2,4,6-trans-octatrienoic acid and ethyl carbonic acid.
